# EUROPEAN PATENT APPLICATION

(11) **EP 4 414 363 A1**
(43) Date of publication of application: **14.08.2024**
(21) Application number: 24156197.6
(22) Date of filing: 07.02.2024
(51) Int. Cl.: C07D 401/04, A61P 25/28, A61K 31/4468, A61K 31/454

(54) **INDOLYLACETIC DERIVATIVES FOR USE IN THE TREATMENT OF NEURODEGENERATIVE DISEASES**

(30) Priority: 08.02.2023 IT 202300002109
(71) Applicant: Miuli Pharma S.r.l., 80035 Nola (NA) (IT)
(72) Inventor: Caliendo, Giuseppe, 80034 Marigliano (NA) (IT); Corvino, Angela, 81100 Caserta (CE) (IT); Fiorino, Ferdinando, 82100 Benevento (BN) (IT); Frecentese, Francesco, 81031 Aversa (CE) (IT); Gargiulo, Michele, 80039 Saviano (NA) (IT); Perissutti, Elisa, 86079 Venafro (IS) (IT); Pignataro, Giuseppe, 80128 Napoli (NA) (IT); Santagada, Vincenzo, 80127 Napoli (NA) (IT); Severino, Beatrice, 80018 Mugnano di Napoli (NA) (IT)
(74) Representative: Dragotti & Associati S.R.L.

(57) **Abstract**

The present invention relates to indolylacetic derivatives of Formula (I) for use in the treatment of neurodegenerative diseases, preferably Amyotrophic Lateral Sclerosis (ALS). The invention further relates to pharmaceutical compositions containing the same, as well as a process for obtaining the indolylacetic derivatives.

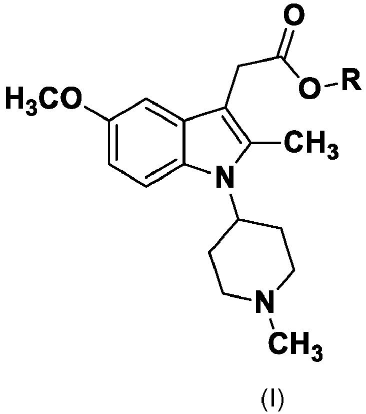

## Description

### FIELD OF THE INVENTION

The present invention relates to indolylacetic derivatives of Formula (I) for use in the treatment of neurodegenerative diseases, preferably Amyotrophic Lateral Sclerosis (ALS). The invention further relates to pharmaceutical compositions containing the same, as well as a process for obtaining the indolylacetic derivatives.

### BACKGROUND OF THE INVENTION

Bicyclic heterocyclic structures are commonly found both in natural and synthetic biologically relevant compounds with indole are the most widespread scaffold of this type. In nature, the indole nucleus may be found in neurotransmitters and autacoids such as serotonin, melatonin, and melanin, which derive from the corresponding amino acid tryptophan, and in a plethora of alkaloids. Dozens of synthetic routes have been developed to afford thousands of compounds based on the indole scaffold [1,2] and more than 300 indole-based small molecules can be found in DrugBank [3]. Over the years, indole has proved to be a versatile scaffold for the design of molecules acting as anti-inflammatory agents such as indomethacin, acemetacin and etodolac.

Amyotrophic lateral sclerosis (ALS) is a progressive and devastating neurodegenerative disease characterized by the loss of Motor Neurons (MNs) in spinal cord, motor cortex and brainstem [4]. Usually, the disease shows a peak of onset around 45-60 years and has a post-diagnosis survival time of approximately 3 to 5 years. Nonetheless, ALS is a clinically heterogeneous pathology, and some patients survive longer and reveal a less aggressive disease [5, 6]. Clinically, the loss of motor neurons is associated to progressive muscle weakening and fasciculation.

In the later disease stages, the patients become paralyzed. In addition, up to 50% of patients can show cognitive impairment and mild memory decline. Ultimately, ALS induces paralysis and premature death which is generally caused by respiratory failure [7-9].

The neuropathological hallmarks of this neuromuscular disorder are degeneration of motor neurons in the spinal anterior horn and motor cortex and loss of axons in the lateral columns of the spinal cord [10].

Based on the inheritance of the disease, ALS is classified in two forms: the sporadic form (sALS) that includes the majority of ALS cases and, the familiar form (fALS) that regards approximately 5-10% of cases [11-12].

The neuroinflammatory component of ALS is being characterized and include the overexpression of mediators such as iNOS and TNF-α. In addition, infiltration of lymphocytes and macrophages, activation of microglia and reactive astrocytes, as well as the involvement of complement have been extensively described [13].

Currently, there are no effective treatment for ALS. In fact, the NMDA blocker riluzole and the ROS scavenger edaravone represent the only two drugs approved for ALS, but these drugs prolong life expectancy for less than 3 months [14].

Two 2-methyl-3-indolylacetic derivatives, hereinafter identified as compounds II (R=H in Formula I) and III (R=-CH₂CH₃ in Formula I) have been published as COX-1 inhibitors with analgesic and anti-inflammatory *in vivo* properties [15]:

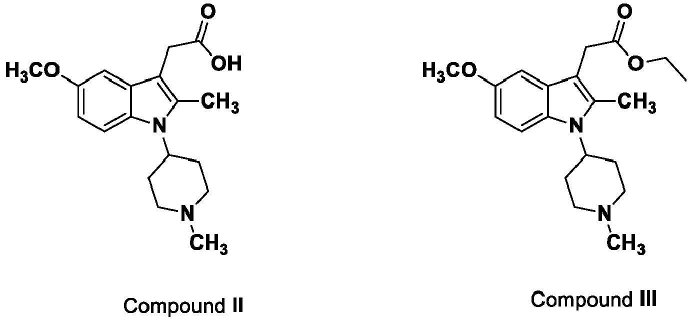

The analgesic activities were comparable to indomethacin, while in addition and most importantly, both compounds appeared to be devoid of gastric effects (gastric damage evaluated as the lengths of the lesions in millimeters [15]; indomethacin 50 ± 8 mm vs compound III 3 ± 0.6 mm and compound II 0 mm).

In the present invention compounds II and III are described for use in the treatment of neurodegenerative diseases, preferably for use in the treatment of ALS.

### SUMMARY OF THE INVENTION

Amyotrophic lateral sclerosis (ALS) is a progressive and devastating neurodegenerative disease characterized by the loss of Motor Neurons (MNs) in spinal cord, motor cortex and brainstem.

The neuroinflammatory component of ALS is being characterized and include the overexpression of mediators such as iNOS and TNF-α.

Currently, there are no effective treatment for ALS. In fact, the NMDA blocker riluzole and the ROS scavenger edaravone represent the only two drugs approved for ALS, but these drugs prolong life expectancy for less than 3 months.

The present inventors have surprisingly found that the compounds of the invention, of Formula (I) are able to slowdown the progression of motor neurons disorders characterized by a strong neuroinflammatory component including Amyotrophic Lateral Sclerosis (ALS) without interfering with COX-2 expression.

The present inventors have also surprisingly found that the combination of the essential parts of two small molecules, namely the opioids containing a 4-piperidinyl ring with the known NSAID indomethacin, might lead to new medicaments for the treatment of neurodegenerative diseases, preferably Amyotrophic Lateral Sclerosis (ALS).

Accordingly, a first object of the present invention is a compound having an indolylacetic structure of Formula (I) or pharmaceutically acceptable salts thereof, for use in the treatment of a neurodegenerative disease.

The second object of the present invention is a pharmaceutical composition comprising an indolylacetic derivative of Formula (I) or pharmaceutically acceptable salts thereof, in combination with at least one physiologically acceptable excipient, for use in the treatment of a neurodegenerative disease.

The third object of the present invention is a process for the preparation of the indolylacetic derivatives of Formula (I) as described above or pharmaceutically acceptable salts thereof, which comprises the following steps:
a) reacting p-anisidine with 1-methylpiperidin-4-one followed by applying microwave irradiation to obtain N-(1-Methyl-4-piperidinyl)-4-anisidine;
b) reacting N-(1-Methyl-4-piperidinyl)-4-anisidine with NaNO₂ to obtain N-(1-Methyl-4-piperidinyl)-4-anisylhydrazine;
c) reacting N-(1-Methyl-4-piperidinyl)-4-anisylhydrazine with ethyl levulinate followed by applying microwave irradiation to obtain a derivative of Formula (I);
d) optionally, salifying the derivative of Formula (I).

### DEFINITIONS

Unless otherwise defined, all terms of art, notations and other scientific terminology used herein are intended to have the meanings commonly understood by those of skill in the art to which this disclosure pertains. In some cases, terms with commonly understood meanings are defined herein for clarity and/or for ready reference; thus, the inclusion of such definitions herein should not be construed to represent a substantial difference over what is generally understood in the art.

The term "physiologically acceptable excipient" herein refers to a substance devoid of any pharmacological effect of its own and which does not produce adverse reactions when administered to a mammal, preferably a human. Physiologically acceptable excipients are well known in the art and are disclosed, for instance in the Handbook of Pharmaceutical Excipients, sixth edition 2009, herein incorporated by reference.

The term "pharmaceutically acceptable salts thereof" herein refers to those salts which possess the biological effectiveness and properties of the salified compound, and which do not produce adverse reactions when administered to a mammal, preferably a human. The pharmaceutically acceptable salts may be inorganic or organic salts; examples of pharmaceutically acceptable salts include but are not limited to: carbonate, hydrochloride, hydrobromide, sulphate, hydrogen sulphate, citrate, maleate, fumarate, trifluoroacetate, 2-naphthalenesulphonate, and paratoluenesulphonate. Further information on pharmaceutically acceptable salts can be found in Handbook of pharmaceutical salts, P. Stahl, C. Wermuth, WILEY-VCH, 127-133, 2008, herein incorporated by reference.

The terms "approximately" and "about" herein refer to the range of the experimental error, which may occur in a measurement.

The terms "comprising", "having", "including" and "containing" are to be construed open-ended terms (i.e. meaning "including, but not limited to") and are to be considered as providing support also for terms as "consist essentially of", "consisting essentially of", "consist of" or "consisting of".

The terms "consist essentially of", "consisting essentially of" are to be construed as semi-closed terms, meaning that no other ingredients which materially affects the basic and novel characteristics of the invention are included (optional excipients may thus be included).

The terms "consists of", "consisting of" are to be construed as closed terms.

The term "C₁-C₄ alkyl" refers herein to a branched or linear hydrocarbon containing 1 to 4 carbon atoms. Examples of C₁-C₄ alkyl groups include, but are not limited to, methyl, ethyl, n-propyl, isopropyl, n-butyl, sec-butyl, isobutyl, tert-butyl; preferably methyl, ethyl, n-propyl, isopropyl.

The term "4-substituted phenyl ring" refers herein to substitution with a moiety that is chemically allowed and characterized by a phenyl ring bearing in position 4 a thioamidic, isothiocyanic, dithioesteric or 3H,1,2-dithiole-3-thionic group. Examples of preferred 4-substituted phenyl rings include but are not limited to benzothioamide, isothiocyanatobenzene, 5-phenyl-3H-1,2-dithiole-3-thione, ethyl benzodithioate, 5-phenyl-3H-1,2,4-dithiazol-3-one.

### DESCRIPTION OF THE FIGURES

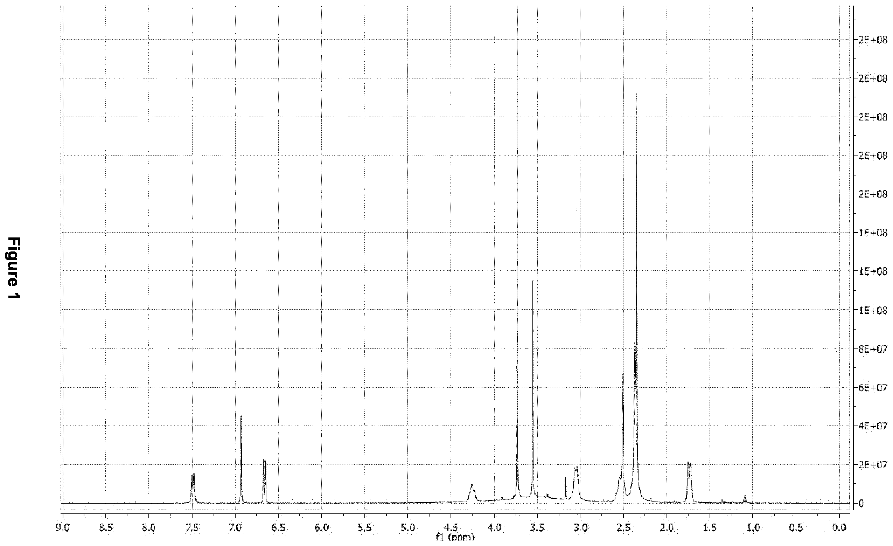

**Figure 1** shows the ¹H-NMR (600 MHz; DMSO-d6) spectrum of Compound **II.**

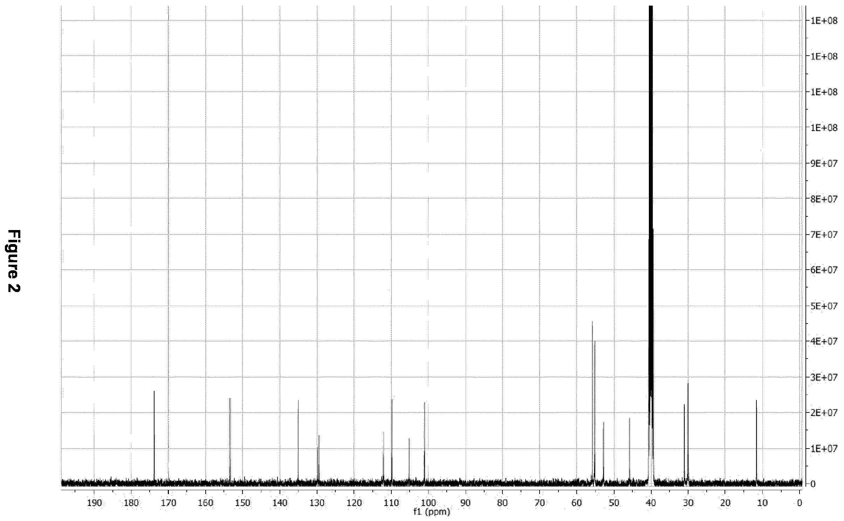

**Figure 2** shows the ¹³C-NMR (150 MHz; DMSO-*d₆*) spectrum of Compound **II.**

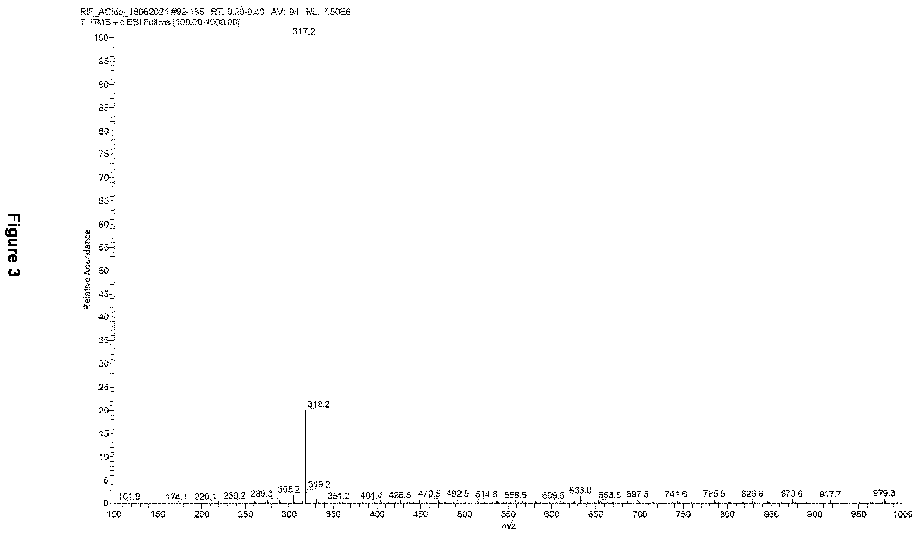

**Figure 3** shows the ESI-MS spectrum (full scan; positive ionization mode) of Compound **II.**

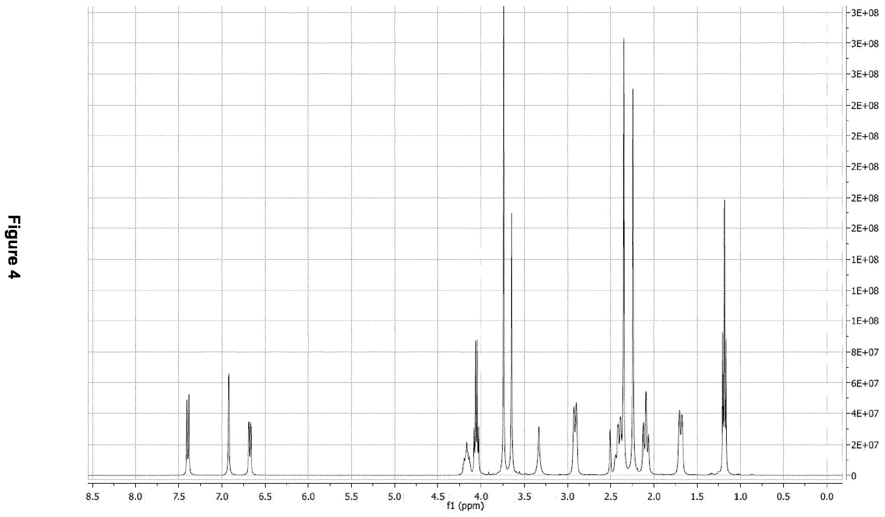

**Figure 4** shows the ¹H-NMR (600 MHz; DMSO-d6) spectrum of Compound **III.**

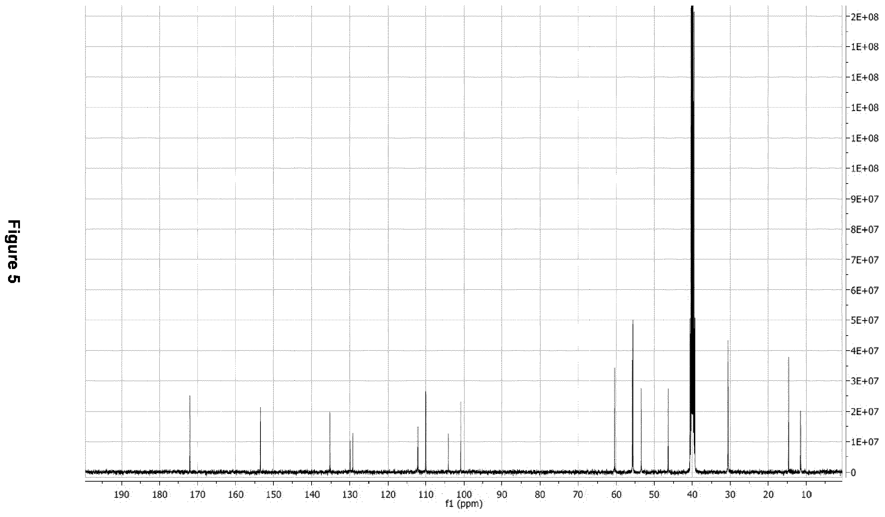

**Figure 5** shows the ¹³C-NMR (150 MHz; DMSO-*d₆*) spectrum of Compound **III.**

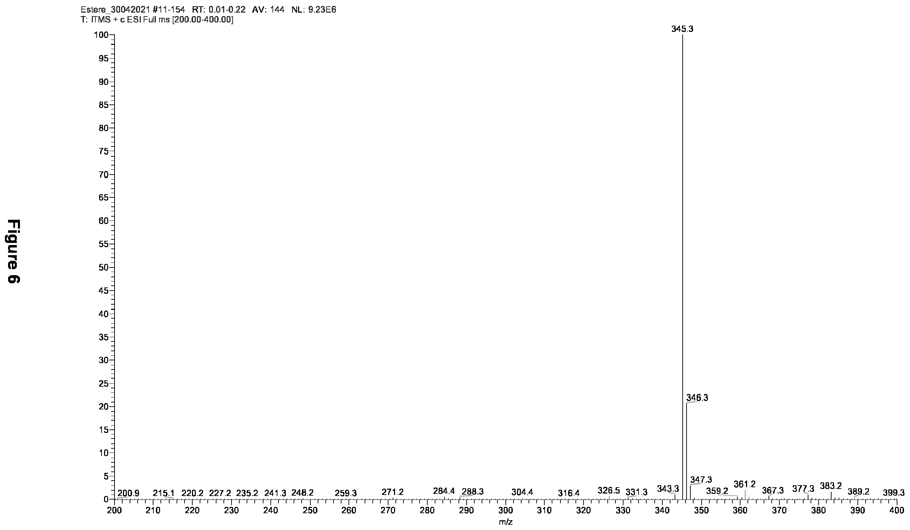

**Figure 6** shows the ESI-MS spectrum (full scan; positive ionization mode) of Compound **III.**

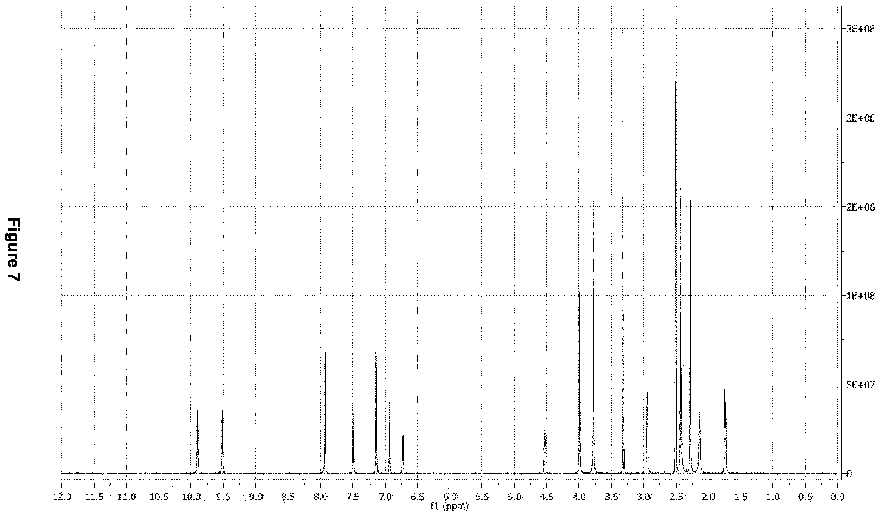

**Figure 7** shows the ¹H-NMR (600 MHz; DMSO-d6) spectrum of Compound **IV.**

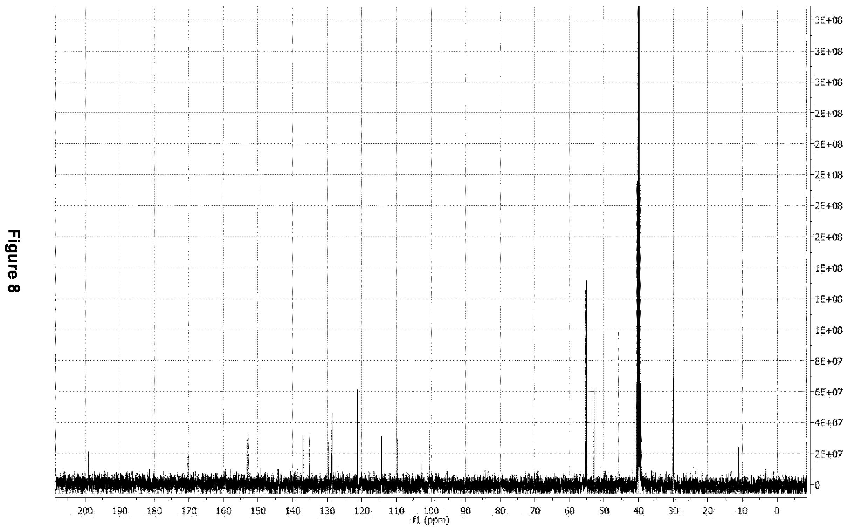

**Figure 8** shows the ¹³C-NMR (150 MHz; DMSO-*d₆*) spectrum of Compound **IV.**

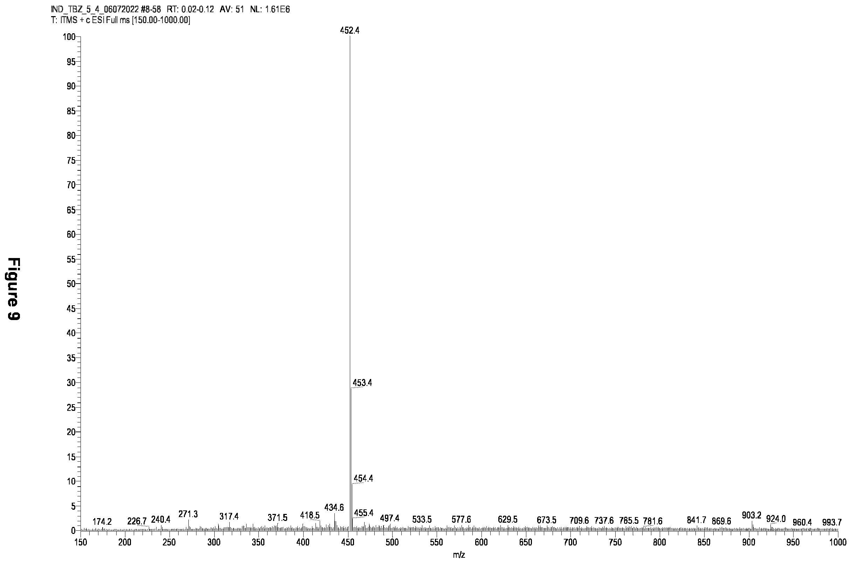

**Figure 9** shows the ESI-MS spectrum (full scan; positive ionization mode) of Compound **IV.**
**Figure 10** shows the enhanced survival of neuronal cells exposed to the neurotoxin L-BMAA induced by compounds **II-IV.**
**Figure 11** shows the effect of Compounds **II-IV** on LPS-induced iNOS expression in microglia cells.
**Figure 12** shows the effect of Compounds **III** on LPS-induced TNF-α expression in microglia cells.
**Figure 13** shows the lack of effects on COX2 expression by all considered compounds.
**Figure 14** shows the effect of prolonged compound **III** treatment on motor symptoms and survival rate in ALS affected mice (SOD1 G93A).

### DESCRIPTION OF THE INVENTION

The invention is related to compounds having a general Formula (I) or pharmaceutically acceptable salts thereof: 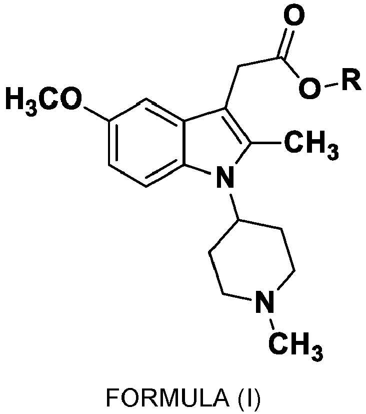 wherein R is a hydrogen atom, a C₁-C₄ alkyl group or 4'-substituted phenyl group, for use in the treatment of a neurodegenerative disease.

The alkyl group is preferably represented by an ethyl group.

Preferably, the 4'-substituted phenyl group is selected from benzothioamide, isothiocyanatobenzene, 5-phenyl-3H-1,2-dithiole-3-thione, ethyl benzodithioate, 5-phenyl-3H-1,2,4-dithiazol-3-one. The 4'-substituted phenyl ring is more preferably represented by benzothioamide.

Preferably, the neurodegenerative disease is selected from the group consisting of ALS, Spinal Muscular Atrophy (SMA), Alzheimer's disease, Huntington's disease, Parkinson's disease, multiple sclerosis and other neurodegenerative pathologies (i. e. Spinal Bulbar Muscular Atrophy (SBMA)). More preferably, ALS.

According to a preferred embodiment, the compounds of Formula (I) are selected from compounds II, III and IV.

Compound IV was synthesized starting from compound II; It has been designed and synthesized (Scheme 1) as molecular hybrid with 4-hydroxythiobenzamide:

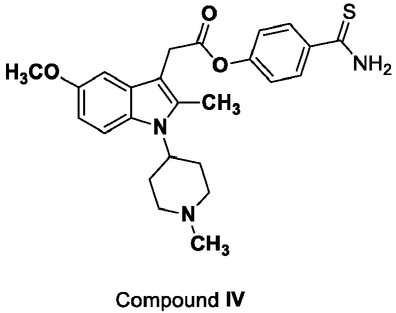

The gaseous neurotransmitter H₂S shows an effect against oxidative stress, and the consequent neuroinflammation, by inhibiting the release of proinflammatory factors in the CNS. Neuroinflammation is considered as a key factor in neurodegeneration in fact inflammatory processes have been described in many neurodegenerative diseases, including Parkinson's disease (PD), Alzheimer's disease (AD), amyotrophic lateral sclerosis (ALS), and multiple sclerosis (MS). For this reason, many therapeutics are aimed at delaying or stopping advancement of inflammation in neurodegenerative diseases [16].

H2S provides an antioxidant function through elevation of CBS [17]. Several studies have reported that H₂S exerts anti-inflammatory and anti-apoptotic effects in CNS [18], thus supporting H₂S role in PD, ALS, MS, and a growing number of other CNS pathologies. Neuroprotective effects of H₂S donors (i.e., NaHS and Na₂S) have been reported *in vitro* and *in vivo* in a variety of animal models [19-20].

Compounds **II-IV** according to the invention showed a significant effect in the control of cell death induced *in vitro* by the exposure of SH-SY5Y neuronal cell lines to the neurotoxin L-BMAA. As for the mechanism of action, compound **II** and compound **IV** were able to prevent iNOS expression induced in BV2 microglial cells by LPS exposure. Compound **III** was able to prevent TNF- α expression but not COX2, induced in BV2 microglial cells by LPS exposure. Notably, prolonged compound **III** treatment ameliorated motor symptoms and increased survival rate in ALS mice (SOD1 G93A).

For the purpose of the present invention, the indolylacetic derivatives of Formula I according to the present invention are formulated in pharmaceutical compositions suitable for use in the treatment of neurodegenerative diseases, preferably in the form of powder, suspension or solution, more preferably said compositions are administered by oral route. The pharmaceutical compositions can be prepared according to conventional methods, for example as disclosed in Remington, "The Science and Practice of Pharmacy", 21st ed. (Lippincott Williams and Wilkins).

### EXPERIMENTAL SECTION

### MATERIALS AND METHODS

All the commercial reagents and solvents have been purchased from Merck-Sigma Aldrich. Reactions were stirred at 400 rpm by Heidolph MR Hei-Standard magnetic stirrer. Solutions were concentrated with a Buchi R-114 rotary evaporator at low pressure. All reactions were followed by TLC carried out on Merck silica gel 60 F254 plates with fluorescent indicator on the plates and were visualized with UV light (254 nm). Preparative chromatographic purifications were performed using silica gel column (Kieselgel 60).

Microwave reactions were performed using a microwave oven (ETHOS 1600, Milestone^{®}) especially designed for organic synthesis. Identification of the optimum profile power/time and temperature for the synthesis was preliminarily conducted. The temperature of the stirred reaction mixture was monitored directly by a microwave-transparent fluoroptic probe inserted into the solution.

Melting points, determined using a Buchi Melting Point B-540 instrument, are uncorrected and represent values obtained on re-crystallized or chromatographically purified material. Mass spectra of final products were performed on LTQ Orbitrap XL^{™} Fourier transform mass spectrometer (FTMS) equipped with an ESI ION MAX^{™} (Thermo Fisher, San José, USA) source operating in positive mode. NMR experiments were recorded on Varian Mercury Plus 600 MHz instrument. All spectra were recorded in DMSO-d6. Chemical shifts are reported in ppm. The following abbreviations are used to describe peak patterns when appropriate: s (singlet), d (doublet), dd (double doublet), q (quartet), m (multiplet).

### CHEMISTRY

For the preparation of compounds **II-IV** the present inventors followed with modifications the procedures adopted in literature [15]. The synthetic route is reported in Scheme 1. Microwave irradiation proved to increase the yields and reduced the reaction times for the preparation of intermediates **1** and **3.** Briefly, the aniline derivative **1** was obtained by a reductive amination of p-anisidine with 1-methylpiperidin-4-one in presence of sodium cyanoborohydride in CH₃OH containing 1% of acetic acid by microwave irradiation. The nitrosation followed by a reduction step with LiAlH₄ afforded the hydrazine **2.** The Fischer indole reaction of hydrazine **2** was performed as a "one-pot" reaction under acidic conditions by flash microwave irradiation yielding compound **III** in very high yield 92% and short irradiation time (55') (Scheme 1). Starting from compound **III,** compound **II** was obtained by ethyl ester saponification. Coupling of this latter with 4-OH-thiobenzamide by EDC gave the desired ester corresponding to compound **IV.**

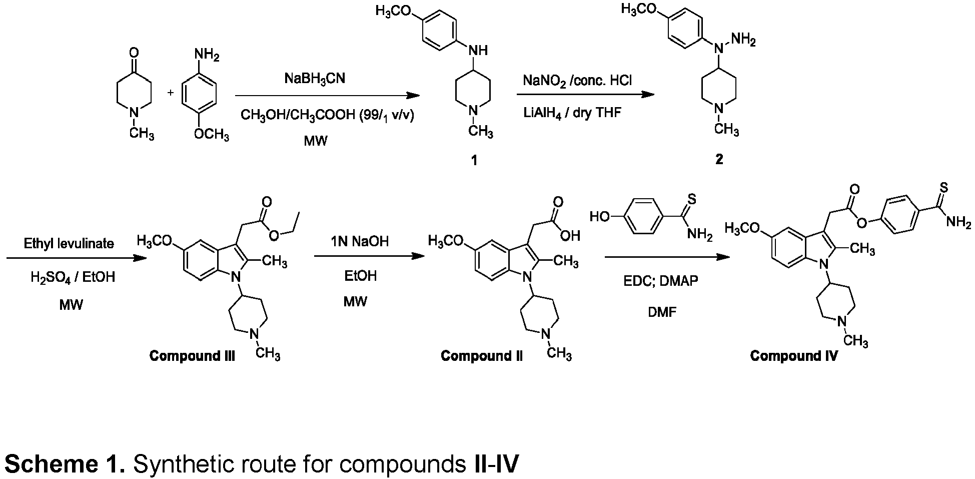

### EXAMPLE 1

### N-(1-Methyl-4-piperidinyl)-4-anisidine (1)

p-Anisidine (10 g, 0.081 mol) and 1-methylpiperidin-4-one (7.1 g, 0.063 mol) were dissolved in a mixture of CH₃OH/CH₃COOH (60 mL, 99:1, v/v) and were stirred for 10 min at room temperature. NaBH₃CN (2.58 g, 0.041 mol) was dissolved in methanol (10 mL) and once added to the reaction the mixture was heated by microwave (1h, 45°C, 300W). The solvent was evaporated, and the crude material was then dissolved in ethyl acetate (350 mL) and washed with brine (3 × 50 mL) and water (50 mL). The organic layer was dried on anhydrous Na₂SO₄ and filtered, and the solvent was evaporated. After chromatography on a silica gel column (eluent, 6:4 dichloromethane/methanol) 13,04 g of **1** was obtained (95%) as an oil.

### EXAMPLE 2

### N-(1-Methyl-4-piperidinyl)-4-anisylhydrazine (2)

N-(1-Methyl-4-piperidinyl)-4-anisidine **(1,** 6.5 g, 0.03 mol) in absolute ethanol (70 mL) was cooled to 0 °C and kept under stirring. 37% HCl (63,3 mL) was added slowly. A solution of NaNO₂ (2.64 g, 0.04 mol) in H₂O (15 mL) was added dropwise, keeping the mixture below 0 °C. The mixture was further stirred at 0°C for 3 h. Water (200 mL) was then added and treated with 6N NaOH until pH was alkaline. The solution was extracted with ethyl acetate (3 × 200 mL), washed with water, dried on anhydrous Na₂SO₄, and filtered, and the solvent was evaporated. The crude nitroso compound was used without further purification. A suspension of LiAlH₄ (3.2 g) in THF (28 mL) was heated to reflux. The crude nitroso compound in THF was added dropwise. The mixture was stirred at reflux for 1 h and then cooled to room temperature. Successively, water (10 mL) and aqueous 3N NaOH (10 mL) were added dropwise. The mixture was filtered on Celite, and the residue was washed several times with dichloromethane. The solvent was removed, obtaining 6.1 g of **2** (88%).

### EXAMPLE 3

### Ethyl 2-(5-methoxy-2-methyl-1-(1-methylpiperidin-4-yl)-1H-indol-3-yl)acetate (Compound III)

N-(1-Methyl-4-piperidinyl)-4-anisylhydrazine **2** (4.7 g, 0.020 mol) and ethyl levulinate (2.88 g, 0.020 mol) were dissolved into absolute ethanol (80 mL) and were heated by microwave in a closed vessel at 78°C for 40 min (400W). After evaporation of the solvent, ethyl acetate (80 mL) was added. The organic phase was washed with brine, dried on anhydrous Na₂SO₄, and filtered, and the solvent was evaporated. Sulfuric acid (96%, 1 mL) was added to a residue dissolved in absolute ethanol, and the mixture was heated again by microwave (closed vessel, 400W, 78°C) for 15 min. Then the solution was made alkaline with 1 N NaOH. Ethanol was removed and the extraction performed with ethyl acetate (3 × 150 mL). The organic layers were washed with brine, dried on anhydrous Na₂SO₄, and filtered, and the solvent was evaporated. After chromatography on silica gel column (eluent, 94:5:1 dichloromethane/methanol/NH₄OH), a yield of 6.4 g of compound **III** was obtained (92%): mp 68-70 °C
¹H-NMR (600 MHz, DMSO-d6) δ 7.39 (d, 1H, J=8.9 Hz), 6.93 (d, 1H, J = 2.1 Hz), 6.66 (dd, 1H, J=8.9 Hz, 2.1 Hz), 4.16 (m, 1H), 4.06 (q, 2H, J=6.9 Hz), 3.74 (s, 3H), 3.62 (s, 2H), 2.91 (m, 2H), 2.36 (m, 5H), 2.24 (s, 3H), 2.10 (m, 2H), 1.69 (m, 2H), 1.18 (t, 3H, J=6.9 Hz).

¹³C-NMR (150 MHz, DMSO-d6) δ 172.0, 153.4, 135.3, 129.9, 129.2, 112.1, 110.0, 104.1,100.8, 60.4, 55.8, 55.6, 53.4, 46.4, 30.6, 30.5, 14.6, 11.5. ESI-MS: 345.2 [M+H]⁺.

### EXAMPLE 4

### 2-(5-methoxy-2-methyl-1-(1-methylpiperidin-4-yl)-1H-indol-3-yl)acetic acid (Compound II)

To a solution of ethyl 2-(5-methoxy-2-methyl-1-(1-methylpiperidin-4-yl)-1H-indol-3-yl)acetate **(III,** 4 g, 0.011 mol) in ethanol (70 mL), an amount of 40 mL of 1N NaOH was added. The mixture was heated by microwave for 3 min (78°C, 500W). Then the solution was neutralized with 1N HCl. Ethanol was removed and the extraction performed with ethyl acetate (3 × 150 mL). The organic layers were washed with brine, dried on anhydrous Na₂SO₄, and filtered, and the solvent was evaporated, yielding 3.4 g of compound **II** (98%) as a white solid: mp 172-173 °C

¹H-NMR (600 MHz, DMSO-*d6*) δ 7.49 (d, 1H, J=8.9 Hz), 6.93 (d, 1H, J = 2.1 Hz), 6.66 (dd, 1H, J=8.9 Hz, 2.1 Hz), 4.25 (m, 1H), 3.73 (s, 3H), 3.55 (s, 2H), 3.05 (m, 2H), 2.54 (m, 2H), 2.36 (m, 5H), 2.35 (s, 3H), 1.73 (m, 2H).

¹³C-NMR (150 MHz, DMSO-d6) δ 173.8, 153.3, 135.0, 129.8, 129.4, 112.0, 109.8,105.1,101.1, 55.8, 55.2, 52.8, 45.8, 31.0, 30.0, 11.5. ESI-MS: 317.2 [M+H]⁺.

### EXAMPLE 5

### 4-carbamothioylphenyl-2-(5-Methoxy-2-methyl-1-(1-methylpiperidin-4-yl)-1H-indol-3-yl)acetate (Compound IV)

[5-methoxy-2-methyl-1-(1-methylpiperidin-4-yl)-1H-indol-3-yl]-acetic acid (**II,** 1 g, 0.00316 mol) was dissolved in dimethylformamide (10 mL); EDAC (0.67g, 0.00340 mol) and DMAP (0.040g, 0.000316 mol) were added and the reaction mixture was kept under stirring for 10 min at a temperature of 0°C. Then, 4-hydroxythiobenzamide (0.73g, 0.00474 mol) was added to the mixture and the reaction was kept under stirring at room temperature overnight. The solvent was removed by distillation under reduced pressure, and the residue was purified by silica gel column chromatography (dichloromethane/methanol, 8:2, v/v) giving 630 mg of compound **IV.** Yield 44%. Yellow solid. M.p.: 145-146°C.

¹H-NMR (600 MHz, DMSO-*d6*) δ 9.90 (s, 1H), 9.52 (s, 1H), 7.93 (d, 2H, J = 8.6 Hz), 7.49 (d, 1H, J=8.9 Hz), 7.14 (d, 2H, J = 8.6 Hz), 6.93 (d, 1H, J = 2.1 Hz), 6.73 (dd, 1H, J=8.9 Hz, 2.1 Hz), 4.53 (m, 1H), 3.99 (s, 2H), 3.78 (s, 3H), 2.94 (m, 2H), 2.43 (s, 3H), 2.42 (m, 2H), 2.28 (s, 3H), 2.14 (m, 2H), 1.73 (m, 2H).

¹³C-NMR (150 MHz, DMSO-*d6*) δ 199.0, 170.2, 153.1, 152.8, 137.0, 135.2, 129.7, 128.8, 128.7, 121.2, 114.3, 109.7,102.9,100.4, 55.4, 55.1, 52.9, 45.9, 30.0, 29.9, 11.1. ESI-MS: 452.3 [M+H]⁺.

### Cell lines

Human neuroblastoma SH-SY5Y cells (Cell bank Line Collection ICLC) and immortalized murine microglial BV-2 cell line were grown as monolayers in polystyrene dishes (12 or 6 wells plate) in Dulbecco's modified Eagle's medium (DMEM) with 10% (v/v) fetal bovine serum (FBS), L glutamine (2 mM), 1% penicillin (50 IU/ml) and streptomycin (50 µg/ml). Cells were grown in a humidified atmosphere containing 95% air and 5% CO₂, and the medium was changed every two days. All experiments were conducted in cultures containing cells between the 4th and 8th passages.

SH-SY5Y and BV-2 cells were plated with a confluence of 65.000 cell/mL. After 24 hours of cell seeding, medium was changed for 6h starvation in a medium containing 0% FBS.

SH-SY5Y cells were exposed to the cyanobacterial toxin β-N-methylamino-L-alanine (L-BMAA) at 2 mM for 120 h in a medium containing 0% FBS. The neurotoxin was replaced at day 1, 3 and 5 while indolylacetic derivatives **II, III** and **IV** (1,10, 100 nM and 1 uM) were added to the cell culture medium 20 min before each L-BMAA exposure.

BV-2 cells were exposed to bacterial toxins Lipopolysaccharides (LPS) at 100 ng/mL and indolylacetic derivatives **II, III** and **IV** (100nM) for 24h in a medium containing 0% FBS.

Cells were exposed to compounds or vehicle and, after 24 hours, have been analyzed for Western Blotting experiments or MTT assay.

### Determination of cell viability

Cell viability was revealed as previously described [21] by using the MTT (Sigma-Aldrich, Milan Italy) staining. Specifically, after 24 h of treatment, the medium was removed, and cells were incubated in 0.5 mg/mL of MTT solution for 1 hours at 37 °C. The incubation was stopped by adding 500 µL of acidified isopropanol to solubilize the formazan salt, and viability was read by measuring the absorbance at 540 nm with a spectrophotometer.

### Western blot analysis

Western blot analysis was performed as previously described [21]. Protein samples (50 µg) were analyzed on 8% subjected to SDS-polyacrylamide gel electrophoresis (SDS-PAGE) and electrotransferred onto Hybond ECL nitrocellulose paper (Amersham, Milan, Italy). Membranes were blocked with 3% BSA in 0.1% Tween-20 (TBS-T; 2mM Tris-HCl, 50 mM NaCl, pH 7.5) for 1h at RT and subsequently incubated overnight at 4 °C in the blocked buffer with antibodies against iNOS (Rabbit monoclonal 1:1000 dilution; abcam), Cox2 (Rabbit polyclonal 1:1000 diluition, byorbyt), TNF-α (Rabbit polyclonal 1:1000 diluition, Cell Signaling) and β-Actin (Mouse monoclonal, 1:10000, Sigma Aldrich).The membranes were washed with 0.1% Tween 20 and incubated with the secondary antibodies for 1h (1:1000; Amersham, Milan, Italy) at room temperature.

Immunoreactive bands were detected using ECL (Amersham). The optical density of the bands (normalized for β-actin) was determined by Chemi-Doc Imaging System (Bio-Rad, Segrate, Italy).

### Animal model

B6SJL-TgN SOD1/G93A(+)1Gur mice expressing high copy number of mutant human SOD1 with a Gly93Ala substitution [SOD1^{G93A}] and B6SJL-TgN (SOD1)2Gur mice expressing wild-type human SOD1 (WT) were obtained from Jackson Laboratories (Bar Harbor, ME, USA). Transgenic animals have been crossed with background-matched B6SJL wild-type female and selective breeding maintained each transgene in the homozygous state. All transgenic mice were identified analyzing extracts from tail tips by staining for SOD1 as previously described [22].

Overall, 24 male and female mice housed under diurnal lighting conditions (12 h darkness/light) were used. The number of female and male mice was balanced among all the experimental groups.

Experiments were performed according to the international guidelines for animal research and approved by the Animal Care Committee of "Federico II" University of Naples, Italy and Ministry of Health, Italy. All efforts were made to minimize animal suffering and to reduce the number of animals used.

### In vivo treatment

The newly synthesized compound **III,** dissolved in distilled water, was intraperitoneally administered daily at a dose of 1 or 10µg/kg, beginning at 2 months of age until 4,5 months of age.

### Evaluation of motor performance

Hind limb grip test was carried out by placing the mouse on a grid (45 cm long × 28 cm large) upside-down (30 cm above a foam pad). The test was performed once a week and the latency to fall off the grid was also measured up to a maximum of 60 s [22].

Motor coordination and balance was assessed using a five-station mouse rotarod apparatus (Ugo Basile; Milan, Italy) as previously described [22]. In each station, the rod was 6 cm in length and 3 cm in diameter. Mice were trained to maintain balance at increasing speed up to a constant speed of 14 rpm for three consecutive trials. The test sessions were conducted by one rotarod trial administered once a week. In this session, the speed of rotation was increased from 4 to 14 rpm over 60 s. Mice had three trials on the rod, and the latencies to fall were measured once a week and then averaged. The maximum latency of 60 s was assigned to the mice that did not fall at all. Weekly evaluation of hind limb paralysis was performed. Hind limb paralysis was scored when the animal dragged one of its hind limbs, and paralysis of a forelimb was scored when the mouse failed to use its forelimbs for walking or righting. Body weight was measured immediately before each session of behavioral tests.

Disease end stage was defined by the inability of mice to right themselves within 20 s when placed on their sides.

### Statistical analysis

Data were evaluated as means ± SEM. Statistically significant differences among means were determined by one-way ANOVA followed by Student-Newman-Keuls/Bonferroni post-hoc test for western blotting, and Behavioral test. The Kaplan-Meier plot was used to evaluate survival, grip, rotarod and paralysis onset. Student's t-test was used for two groups comparison. Statistical significance was accepted at the 95% confidence level (p < 0.05). Statistical analyses were performed by using GraphPad Prism 5.0 (La Jolla, CA, USA). All experiments were carried out in a blinded manner.

### RESULTS

### Indolylacetic derivatives according to the invention enhanced the survival of neuronal cells exposed to L-BMAA and reduced neuroinflammation

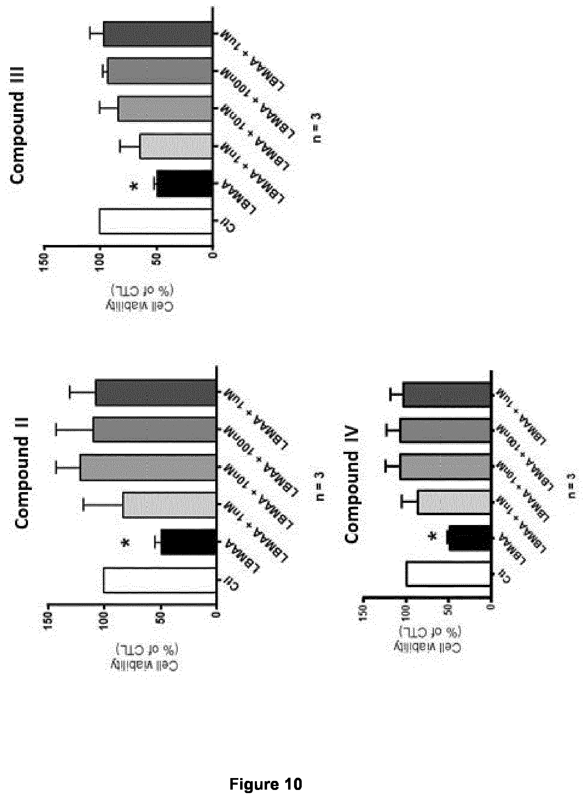

To test whether indolylacetic derivatives protect against L-BMAA-induced toxicity, human SH-SY5Y neuroblastoma cells were exposed to L-BMAA and to compounds **II-IV.** Cell survival was quantified by the MTT assay in: (1) untransfected cells, (2) L-BMAA exposed cells and (3) cells exposed to L-BMAA and different concentrations of the newly synthesized compounds **II-IV** (1,10, 100 nM and 1 uM)). Surprisingly, all derivatives were able to rescue the toxic effect induced by L-BMAA exposure **(****Figure 10****).**

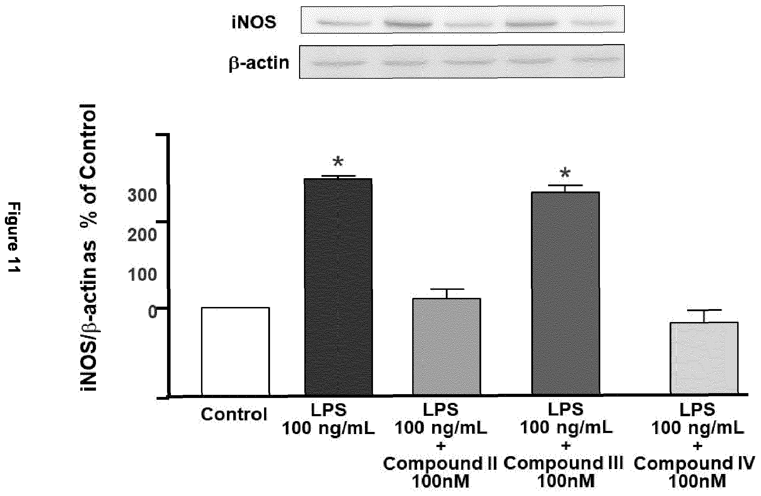

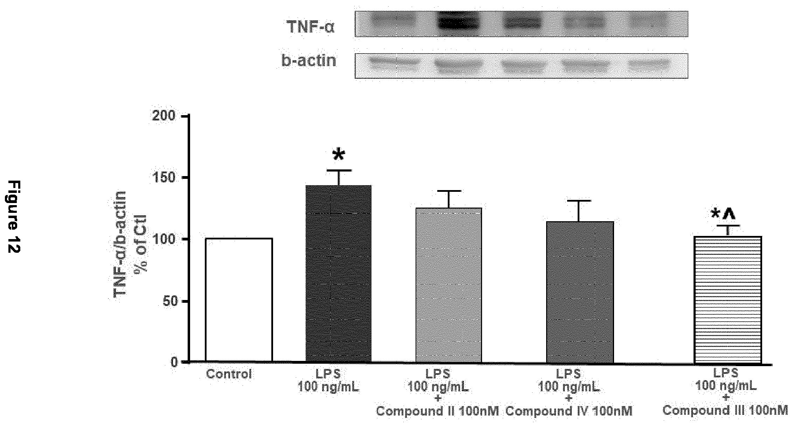

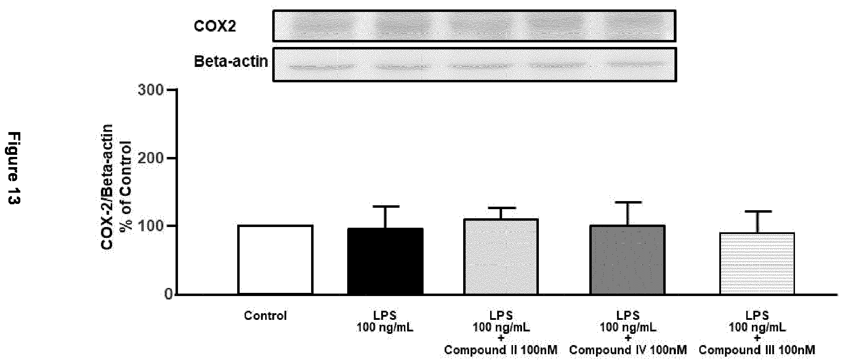

Indolylacetic derivatives **II-IV** according to the invention enhanced the survival of neuronal cells exposed to L-BMAA Furthermore, immunostaining with the inflammation markers COX2, iNOS, and TNF-α antibodies revealed that compounds **II** and **IV** were able to prevent iNOS induction in microglial BV2 cells elicited by LPS **(****Figure 11****),** while compound **III** was able to prevent the overexpression of TNF-α induced by LPS exposure **(****Figure 12****).** All considered compounds did not modify COX2 expression **(****Figure 13****).** Notably, TNF-α represents one of the major mediators of the inflammatory response, being mainly released within the central nervous system (CNS) by reactive astrocytes and microglia. In fact, increased levels of TNFα have been described in plasma, serum, cerebrospinal fluid and CNS tissue from both ALS patients and transgenic animal models of disease.

### Prolonged compound III treatment ameliorated motor symptoms and increased survival rate in SOD1 ^{G93A} mice

To investigate whether the reduced neuroinflammatory response observed *in vitro* through the effect on TNF-α expression was accompanied by an amelioration of the motor performance of these mice, behavioral tests were weekly performed on WT and SOD1^{G93A} mice, starting from the second month after birth (P60), when i.p. administration of compound **III** started and when the mice were asymptomatic. All animals were assigned to three experimental groups: SOD1^{G93A}+vehicle, SOD1 ^{G93A} +compound **III** at 1 mg/kg, and SOD1 ^{G93A} +compound **III** at 10 mg/kg, and then weighed, subjected to rotarod test, grip test and observed to detect the disease onset.

### Survival:

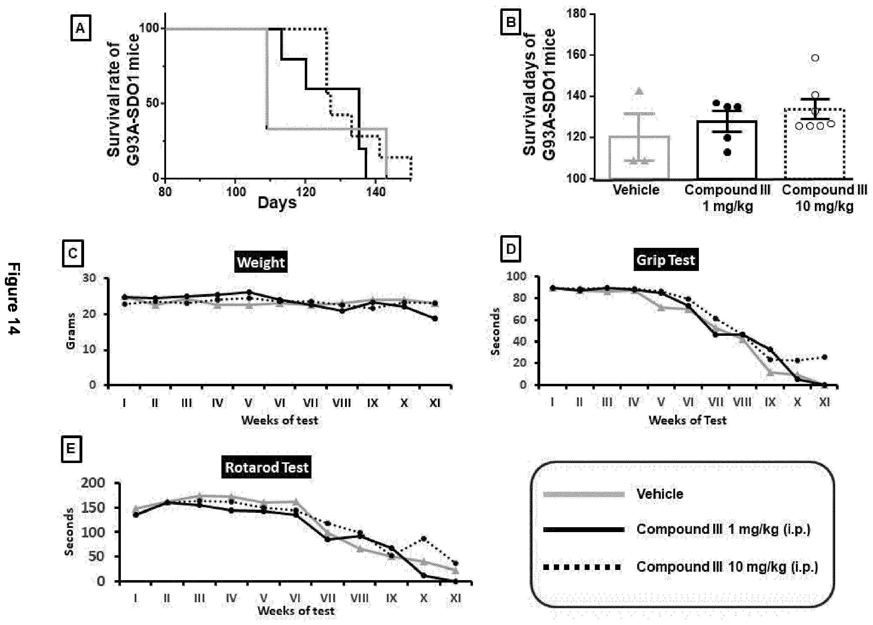

Compound **III** extended the lifespan of ALS mice **(****Figure 14A****).** In fact, the average lifespan of SOD1 ^{G93A} mice treated with vehicle was 127.9 ± 1.65 days, while that of ALS mice treated with compound **III** was significantly longer, being 146.2 ± 3 days **(****Figure 14B****).**

### Paralysis onset

The onset of the disease was defined when denervation-induced muscle atrophy produced 15-20% of the decline in motor performance such as grip strength and rotarod performance. Indeed, the paralysis onset in SOD1^{G93A} treated with vehicle was 33.69 ± 1.31 days while in compound **III** treated SOD1^{G93A} group was 42.47 ± 1.59 days.

### Body Weight

In vehicle SOD1^{G93A} mice the body weight loss was observed at 7 weeks after vehicle treatment, indeed, a 10% reduction in body weight was noted. On the other hand, in the group SOD1^{G93A} treated with compound **III** for 8 weeks the weight remained stable over time. In fact, after 8 weeks of treatment with compound **III,** body weight did not change compared to the group of WT mice. No differences were observed between WT mice treated with compound **III** and WT mice treated with vehicle **(****Figure 14C****).**

### Rotarod test

Motor coordination of WT and G93A mice treated with vehicle or with compound **III** was assessed as the ability of mice to maintain balance on an accelerating rotarod at 1 through 8 weeks of vehicle or compound **III** treatment. The wild type mice were able to maintain balance for the duration of each test and compound **III** treatment did not disrupt this performance. The G93A mice became progressively impaired in motor coordination. There was no statistically significant difference between G93A treatment groups during rotarod training. Significantly, compound **III** treatment blunted the decline in rotarod performance in the G93A mice. Performance of the vehicle treated G93A mice progressed from a slight impairment at 2 weeks of treatment to complete loss of the ability to maintain balance at 8 weeks of treatment (3.7±1 sec). In contrast, performance deteriorated at a slower rate with compound **III** treatment, as reflected in significantly longer durations in the SOD1^{G93A} mice treated with compound **III** compared to vehicle treated animals at weeks 7-8. In fact, at 8 weeks, the compound **III** treated SOD1^{G93A} mice maintained the ability to balance on the rotarod for an average of 21.4±5 sec. The rotarod onset was also delayed in SOD1^{G93A} mice chronically treated with compound **III.** Indeed, 50% of the animals started to fall off the rotarod around the 45^{th} day of compound **III** treatment while the SOD1^{G93A} vehicle treated mice fell around the 30^{th} day **(****Fig.14E****).**

### Grip-strength test

Hindlimb grip test was evaluated by placing the mouse on a grid upside-down and the latency to fall off the grid was measured up to a maximum of 60 s. In the vehicle SOD1^{G93A} mice the loss of limbs strength started evident after 6 weeks of treatment (28.8 ± 4 sec), in fact the grip on the grid decreased by 50% compared to the WT group. In ALS mice treated with compound **III** the grip performance at 6 weeks was 43.8 ± 4 seconds and at 8 weeks the mice remained on the grid for another 20 seconds (F**ig.14D**). Finally, the onset of grip performance was improved in compound **III** G93A mice, 50% of the animals started to lose their grip around the 45^{th} day after treatment while the G93A vehicle mice fell around the 35^{th} day (Fig.14D).

Prolonged compound **III** treatment ameliorated motor symptoms and increased survival rate in SOD1^{G93A} mice.

### CONCLUSIONS

Results described in the present invention support the potentiality of newly synthesized indolylacetic derivatives II-IV in ALS treatment. Indeed, in the attempt to realize an association between two active molecules, the present inventors hypothesized that combination of the essential parts of two small molecules (the opioids containing a 4-piperidinyl ring with the known NSAID indomethacin) might lead to new medicaments for the treatment of Amyotrophic Lateral Sclerosis (ALS). ALS represents a medical need since no effective treatments are present for this progressive and devastating neurodegenerative disease characterized by the loss of Motor Neurons (MNs) in spinal cord, motor cortex and brainstem.

The neuroinflammatory component of ALS is being characterized and, differently from the canonical knowledge in the field attribute to COX activity the main player of this process, it includes the overexpression of mediators such as TNF-α.

Based on that above reported, the present inventors have designed, and synthesized novel substituted 2-methyl-3-indolylacetic derivatives able to prevent ALS-induced TNF-α upregulation. These compounds have been then evaluated for their potential capability of slowing down neurodegeneration associated to ALS progression *in vitro* and *in vivo* preclinical models of the disease.

The newly synthesized compounds II-IV showed a significant effect in the control of cell death evaluated in an *in vitro* model of ALS. More interestingly, the prolonged administration of **III** to ALS mice ameliorated motor symptoms and increased survival rate, thus further supporting the neuroprotective effect of this class of compounds in ALS.

### References

[1] Garg, V.; Maurya, R.K.; Thanikachalam, P.V.; Bansal, G.; Monga, V. An insight into the medicinal perspective of synthetic analogs of indole: A review. Eur. J. Med. Chem. 2019, 180, 562-612.
[2] Kumari, A.; Singh, R.K. Medicinal chemistry of indole derivatives: Current to future therapeutic prospectives. Bioorg. Chem. 2019, 89, 103021.
[3] Wishart, D.S.; Feunang, Y.D.; Guo, A.C.; Lo, E.J.; Marcu, A.; Grant, J.R.; Sajed, T.; Johnson, D.; Li, C.; Sayeeda, Z.; et al. DrugBank 5.0: A major update to the DrugBank database for 2018. Nucleic Acids Res. 2018, 46, D1074-D1082.
[4] Tokuda, E., Furukawa Y. Copper Homeostasis as a Therapeutic Target in Amyotrophic Lateral Sclerosis with SOD1 Mutations. Int. J Mol Sci 2016, 17(5), 636.
[5] Hilton J. B.; White, A. R.; Crouch P. J. Metal-deficient SOD1 in amyotrophic lateral sclerosis. J Mol Med, 2015, 93, 481-487.
[6] Brooks, B. R.; Miller, R. G.; Swash, M.; Munsat, T. L. El Escorial revisited: revised criteria for the diagnosis of amyotrophic lateral sclerosis. Amyotroph. Lateral Scler. Other Motor Neuron Disord. 2000, 1, 293-299.
[7] Lomen-Hoerth, C.; Murphy, J.; Langmore, S.; Kramer, J. H.; Olney, R. K.; Miller, B. Are amyotrophic lateral sclerosis patients cognitively normal? Neurology, 2003, 60(7), 1094-1097.
[8] Rusina, R.; Ridzon, P.; Kulist'ák, P.; Keller, O.; Bartos, A.; Buncová, M.; Fialová, L.; Koukolik, F.; Matěj R. Relationship between ALS and the degree of cognitive impairment, markers of neurodegeneration and predictors for poor outcome. A prospective study. Eur. J. Neurol. 2020, 17, 23-30.
[9] Ringholz, G. M.; Appel, S. H.; Bradshaw, M.; Cooke, N. A.; Mosnik, D. M.; Schulz, P. E. Prevalence and patterns of cognitive impairment in sporadic ALS. Neurology 2005, 65, 586-590.
[10] Saberi, S.; Stauffer, J. E.; Schulte, D. J.; Ravits, J. Neuropathology of amyotrophic lateral sclerosis and its variants. Neurol. Clin. 2015, 33, 855-876.
[11] Wen, X.; Westergard, T.; Pasinelli, P.; Trotti, D. Pathogenic determinants and mechanisms of ALS/FTD linked to hexanucleotide repeat expansions in the C9orf72 gene. Neurosci. Lett. 2017, 636, 16-26.
[12] Katsuno, M.; Tanaka, F.; Sobue, G. Perspectives on molecular targeted therapies and clinical trials for neurodegenerative diseases. J. Neurol. Neurosurg. Psychiatry 2012, 83, 329-335.
[13] Sirabella, R.; Valsecchi, V.; Anzilotti, S.; Cuomo, O.; Vinciguerra, A.; Cepparulo, P.; Brancaccio, P.; Guida, N.; Blondeau, N.; Canzoniero, L. M. T.; Franco, C.; Amoroso, S.; Annunziato, L.; Pignataro G. Ionic Homeostasis Maintenance in ALS: Focus on New Therapeutic Targets Front Neurosci. 2018, 12, 510.
[14] Jaiswal, M. K. Calcium, mitochondria, and the pathogenesis of ALS: the good, the bad, and the ugly. Front. Cell. Neurosci. 2013, 7, 199.
[15] Perissutti, E.; Fiorino, F.; Renner, C.; Severino, B.; Roviezzo, F.; Sautebin, L.; Rossi, A.; Cirino, G.; Santagada, V.; Caliendo, G. Synthesis of 2-methyl-3-indolylacetic derivatives as anti-inflammatory agents that inhibit preferentially cyclooxygenase 1 without gastric damage. J Med Chem. 2006, 49(26), 7774-7780.
[16] Amor, S., Peferoen, L. A. N., Vogel, D.Y.S., Breur, M.; van der Valk, P.; Baker, D.; van Noort J. M. Inflammation in neurodegenerative diseases - an update. Immunology. 2014, 142(2), 151-166.
[17] Tabassum, R.; Jeong, N. Y. Potential for therapeutic use of hydrogen sulfide in oxidative stress-induced neurodegenerative diseases. Int J Med Sci. 2019, 16(10), 1386-1396.
[18] Kida, K.; Ichinose, F. Hydrogen Sulfide and Neuroinflammation. Handb Exp Pharmacol. 2015, 230, 181-9.
[19] Kimura, Y.; Goto, Y. I.; Kimura, H. Antioxidants & Redox Signaling. 2010, 1, 1-13.
[20] Kimura, Y.; Kimura, H. Hydrogen sulfide protects neurons from oxidative stress. FASEB J. 2004, 18(10), 1165-1167.
[21] Laudati, G.; Mascolo, L.; Guida, N.; Sirabella, R.; Pizzorusso, V.; Bruzzaniti, S.; Serani, A.; Di Renzo, G.; Canzoniero, L. M. T.; Formisano, L. Resveratrol treatment reduces the vulnerability of SH-SY5Y cells and cortical neurons overexpressing SOD1-G93A to Thimerosal toxicity through SIRT1/DREAM/PDYN pathway. Neurotoxicology. 2019, 71, 6-15.
[22] Anzilotti, S.; Brancaccio, P.; Simeone, G.; Valsecchi, V.; Vinciguerra, A.; Secondo, A.; Petrozziello, T.; Guida, N.; Sirabella, R.; Cuomo, O.; Cepparulo, P.; Herchuelz, A.; Amoroso, S.; Di Renzo, G.; Annunziato, L.; Pignataro G. Preconditioning, induced by sub-toxic dose of the neurotoxin L-BMAA, delays ALS progression in mice and prevents Na+/Ca2+ exchanger 3 downregulation. Cell Death Dis. 2018, 9(2), 206.

## Claims

1. A compound having an indolylacetic structure of Formula (I), or pharmaceutically acceptable salts thereof: 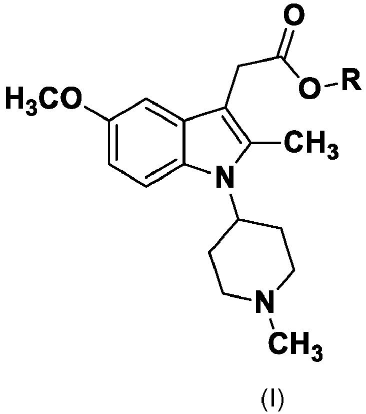 wherein R is a hydrogen atom, a C₁-C₄ alkyl group or 4'-substituted phenyl group, for use in the treatment of a neurodegenerative disease.

2. The compound for use according to claim 1, wherein the 4'-substituted phenyl group is selected from benzothioamide, isothiocyanatobenzene, 5-phenyl-3H-1,2-dithiole-3-thione, ethyl benzodithioate, 5-phenyl-3H-1,2,4-dithiazol-3-one.

3. The compound for use according to claim 1 or 2, selected from compounds **II, III** or **IV:**

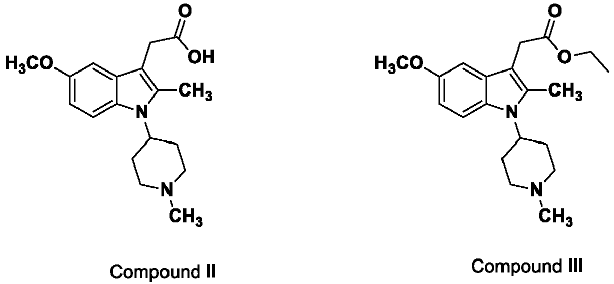

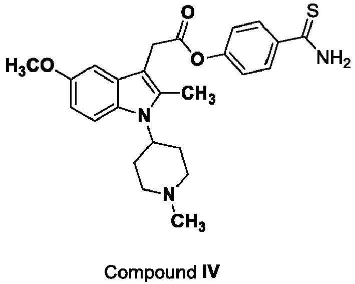

4. The compound for use according to any of the previous claims, wherein the neurodegenerative disease is selected from the group consisting of Amyotrophic Lateral Sclerosis (ALS), Spinal Muscular Atrophy (SMA), Alzheimer's disease, Huntington's disease, Parkinson's disease, multiple sclerosis and other neurodegenerative pathologies, preferably ALS.

5. A pharmaceutical composition comprising an indolylacetic derivative for use according to any of the previous claims, in combination with at least one physiologically acceptable excipient.

6. The pharmaceutical composition for use according to claim 5, wherein said composition is in the form of powder, suspension or solution, preferably said composition is administered by oral route.

7. A process for the preparation of the indolylacetic derivatives of Formula (I) as described in claim 1 or pharmaceutically acceptable salts thereof, which comprises the following steps:
a) reacting p-anisidine with 1-methylpiperidin-4-one followed by applying microwave irradiation to obtain N-(1-Methyl-4-piperidinyl)-4-anisidine;
b) reacting N-(1-Methyl-4-piperidinyl)-4-anisidine with NaNO₂ to obtain N-(1-Methyl-4-piperidinyl)-4-anisylhydrazine;
c) reacting N-(1-Methyl-4-piperidinyl)-4-anisylhydrazine with ethyl levulinate followed by applying microwave irradiation to obtain a derivative of Formula (I);
d) optionally, salifying the derivative of Formula (I).

8. The process according to claim 7, wherein the steps are performed in organic solvent selected from the group consisting of C4-C8 aliphatic esters, C4-C6 cyclic ethers, C1-C4 chlorinated hydrocarbons, aliphatic C2-C4 nitrites, C1-C3 aliphatic amides and mixtures thereof.

9. The process according to claim 8, wherein said organic solvent is selected from the group consisting of tetrahydrofuran, dioxane, ethyl acetate, dichloromethane, chloroform, acetonitrile, dimethylformamide and mixtures thereof.

10. The process according to claim 7, wherein the step a) is performed in the presence of a reagent selected from the group consisting of NaBH₄, NaBH₃CN, and Na(CH₃COO)₃BH.

11. The process according to claim 7, wherein the derivative of Formula (I) is the compound **IV** which is obtained by coupling compound **II** with 4-hydroxythiobenzamide in the presence of a coupling reagent selected from the group consisting of HATU, HBTU, TBTU, DCC, EDAC, CDI, DIPCDI and COMU.
